# EUROPEAN PATENT APPLICATION

(11) **EP 4 101 368 A1**
(43) Date of publication of application: **14.12.2022**
(21) Application number: 21178495.4
(22) Date of filing: 09.06.2021
(51) Int. Cl.: A61B 5/00, G06T 7/00

(54) **DETERMINING SPECULAR REFLECTION INFORMATION**

(71) Applicant: Koninklijke Philips N.V., 5656 AG Eindhoven (NL)
(72) Inventor: Damodaran, Mathivanan, 5656 AE Eindhoven (NL); Palero, Jonathan Alambra, 5656 AE Eindhoven (NL); Varghese, Babu, 5656 AE Eindhoven (NL)
(74) Representative: Philips Intellectual Property & Standards

(57) **Abstract**

A computer-implemented method (100) is described. The method comprises receiving (102) imaging data obtained by an imaging system (204) of a subject (202) illuminated by first illumination (206a) in a first spectral band and second illumination (206b) in a second spectral band comprising different spectral content to the first spectral band. The second illumination incident on the subject is polarized. The imaging system is configured to obtain first imaging data within the first spectral band. The imaging system is further configured to obtain second imaging data within the second spectral band. The imaging system is further configured to admit the first and second illumination into the imaging system depending on a polarization state of reflected first and second illumination received by the imaging system after reflection from a surface of the subject. Information regarding specular reflection from the surface of the subject is determined by comparing the first and second imaging data.

## Description

### FIELD OF THE INVENTION

The invention relates to a method, tangible machine-readable medium and apparatus for use in imaging in certain conditions.

### BACKGROUND OF THE INVENTION

A topic of interest in the field of non-obtrusive measurement and monitoring relates to skin sensing for personal care and health applications. Skin sensing systems are being developed that promise skin quantification and monitoring of features in the skin that may offer users information that is too small to detect, too faint to notice or too slow to follow. To deliver results that are acceptable to users, such skin sensing systems may need to provide sensitivity and specificity when performing skin sensing. Providing measurements taken by such skin sensing systems are proven to be robust and reliable, users may establish trust in these skin sensing systems.

Imaging-based skin sensing systems may implement various imaging techniques in order to determine certain information about the user's skin. Such information may include parameters such as glossiness of the user's skin. Certain systems for determining skin glossiness may be expensive, complex and/or bulky.

### SUMMARY OF THE INVENTION

Aspects or embodiments described herein relate to determining certain information such as glossiness of a surface of a subject. Aspects or embodiments described herein may obviate one or more problems associated with expense, complexity and/or bulkiness of systems for determining such information.

In a first aspect, a method is described. The method is a computer-implemented method. The method comprises receiving imaging data obtained by an imaging system of a subject illuminated by first illumination in a first spectral band and second illumination in a second spectral band comprising different spectral content to the first spectral band. The second illumination incident on the subject is polarized.

The imaging system is configured to obtain first imaging data within the first spectral band by admitting at least part of the first spectral band into the imaging system and preventing admission of at least part of the second spectral band into the imaging system such that a majority of intensity information in the first imaging data obtained within the first spectral band is derived from the first illumination.

The imaging system is further configured to obtain second imaging data within the second spectral band by admitting at least part of the second spectral band into the imaging system and preventing admission of at least part of the first spectral band into the imaging system such that a majority of intensity information in the second imaging data obtained within the second spectral band is derived from the second illumination.

The imaging system is further configured to admit the first and second illumination into the imaging system depending on a polarization state of reflected first and second illumination received by the imaging system after reflection from a surface of the subject such that specular and diffuse reflected first illumination is admitted into the imaging system and diffuse reflected second illumination is admitted into the imaging system.

The method further comprises determining information regarding specular reflection from the surface of the subject by comparing the first and second imaging data.

Some embodiments relating to the first aspect are described below.

In some embodiments, a timeframe over which the first imaging data is obtained at least partially overlaps with a timeframe over which the second imaging data is obtained.

In some embodiments, the imaging system is configured to simultaneously obtain the first and second imaging data.

In some embodiments, the imaging system comprises an imaging system polarizer configured to admit, into the imaging system, reflected first and second illumination that has an electric field component that is parallel to a polarization axis of the imaging system polarizer. The imaging system polarizer is further configured to attenuate reflected first and second illumination that has an electric field component that is perpendicular to the polarization axis.

In some embodiments, the information regarding specular information is indicative of a gloss level of the subject's skin. The gloss level may be determined by calculating a difference between intensity information in the first imaging data and the second imaging data.

In some embodiments, the imaging system comprises a color filter array. The color filter array may be configured to enable at least one imaging device of the imaging system to obtain the first imaging data within the first spectral band and the second imaging data within the second spectral band. The method may further comprise extracting, from raw imaging data obtained by the at least one imaging device, the first imaging data separately from the second imaging data.

In a second aspect, a tangible machine-readable medium is described. The tangible machine-readable medium comprises instructions which, when executed by processing circuitry, causes the processing circuitry to implement the method of the first aspect or any related embodiments.

In a third aspect, apparatus is described. The apparatus comprises processing circuitry. The processing circuitry comprises a receiving module and a determining module.

The receiving module is configured to receive imaging data obtained by an imaging system of a subject illuminated by first illumination in a first spectral band and second illumination in a second spectral band comprising different spectral content to the first spectral band. The second illumination incident on the subject is polarized.

The imaging system is configured to obtain first imaging data within the first spectral band by admitting at least part of the first spectral band into the imaging system and preventing admission of at least part of the second spectral band into the imaging system such that a majority of intensity information in the first imaging data obtained within the first spectral band is derived from the first illumination.

The imaging system is further configured to obtain second imaging data within the second spectral band by admitting at least part of the second spectral band into the imaging system and preventing admission of at least part of the first spectral band into the imaging system such that a majority of intensity information in the second imaging data obtained within the second spectral band is derived from the second illumination.

The imaging system is further configured to admit the first and second illumination into the imaging system depending on a polarization state of reflected first and second illumination received by the imaging system after reflection from a surface of the subject such that specular and diffuse reflected first illumination is admitted into the imaging system and diffuse reflected second illumination is admitted into the imaging system.

The determining module is configured to determine information regarding specular reflection from the surface of the subject by comparing the first and second imaging data.

Some embodiments relating to the third aspect are described below.

In some embodiments, a result of the comparison between the first and second imaging data corresponds to a measure of glossiness of the subject's skin.

In some embodiments, the apparatus further comprises the imaging system and/or an illumination system configured to provide the first and second illumination.

In some embodiments, the imaging system comprises an imaging system polarizer configured to admit, into the imaging system, reflected first and second illumination that has an electric field component that is parallel to a polarization axis of the imaging system polarizer. The imaging system polarizer is further configured to prevent admission, into the imaging system, of reflected first and second illumination that has an electric field component that is perpendicular to the polarization axis.

In some embodiments, the illumination system comprises an illumination system polarizer configured to polarize the second illumination that is directed towards the subject. A polarization axis of the imaging system polarizer may be orthogonal to the polarization axis of the illumination system polarizer.

In some embodiments, the illumination system is configured such that the first illumination directed towards the subject is unpolarized or the illumination system comprises an additional illumination system polarizer configured to polarize the first illumination directed towards the subject such that the polarization state of the first illumination that is directed towards the subject is orthogonal to the polarization state of the second illumination that is directed towards the subject.

In some embodiments, the illumination system is configured to direct the first and second illumination towards the subject such that both a specular and diffuse component of the first and second illumination reflected from the surface of the subject is directed into the imaging system for admission thereinto depending on the polarization state of the reflected first and second illumination.

In some embodiments, the imaging system comprises at least one imaging device and an optical filter array.

The optical filter array may be configured to pass, to a first set of pixels of the at least one imaging device, at least part of the first spectral band into the imaging system such that the majority of intensity information in the first imaging data obtained within the first spectral band is derived from the first illumination.

The optical filter array may be further configured to pass, to a second, different, set of pixels of the at least one imaging device, at least part of the second spectral band into the imaging system such that the majority of intensity information in the second imaging data obtained within the second spectral band is derived from the second illumination.

These and other aspects of the invention will be apparent from and elucidated with reference to the embodiment(s) described hereinafter.

### BRIEF DESCRIPTION OF THE DRAWINGS

Exemplary embodiments of the invention will now be described, by way of example only, with reference to the following drawings, in which:
Fig. 1 refers to a method of determining certain information about a surface of a subject according to an embodiment;
Fig. 2 is a schematic drawing of a system for determining certain information about a surface of a subject according to an embodiment;
Fig. 3 is a schematic drawing of a system for determining certain information about a surface of a subject according to an embodiment;
Fig. 4 depicts example optical parameters of certain components of the system of Fig. 3;
Fig. 5 is a schematic drawing of a representation of the method of determining certain information about a surface of a subject according to an embodiment;
Fig. 6 refers to a method of determining certain information about a surface of a subject according to an embodiment according to an embodiment;
Fig. 7 is a schematic drawing of a machine-readable medium for determining certain information about a surface of a subject according to an embodiment; and
Fig. 8 is a schematic drawing of an apparatus for determining certain information about a surface of a subject according to an embodiment.

### DETAILED DESCRIPTION OF THE EMBODIMENTS

Skin that appears to have a natural, luminous gloss without appearing oily may be desirable in some skin beauty scenarios. The visible appearance of the skin depends on the light interaction with the skin. The level of apparent gloss may depend on the underlying surface and subsurface reflections arising from different angles of light incident on the skin.

The measurement of skin gloss may be of interest in the efficacy testing of certain skincare solutions. For example, skin gloss may be relevant to skin beauty and may have implications for personal confidence and/or achieving a specified appearance.

Skincare products such as make-up, moisturizers, etc., may be applied to the skin, which may affect the apparent gloss of the skin. Further, some devices such as skin cleaning devices, shavers, exfoliators, hydration devices, skin stimulators (e.g., mechanical, electrical, optical) or any other devices which may change the appearance of the skin (e.g., for beauty reasons or otherwise) may also affect the apparent gloss of the skin. Skincare products and/or devices may be used as part of a personal care regime.

Certain personal care regimes may involve characterizing the gloss of the skin (e.g., before and/or after the personal care regime). For example, a user interested in characterizing their skin (e.g., to determine skin gloss) may use certain embodiments described herein to determine information useful for such characterization. This information may be useful for evaluating a personal care regime.

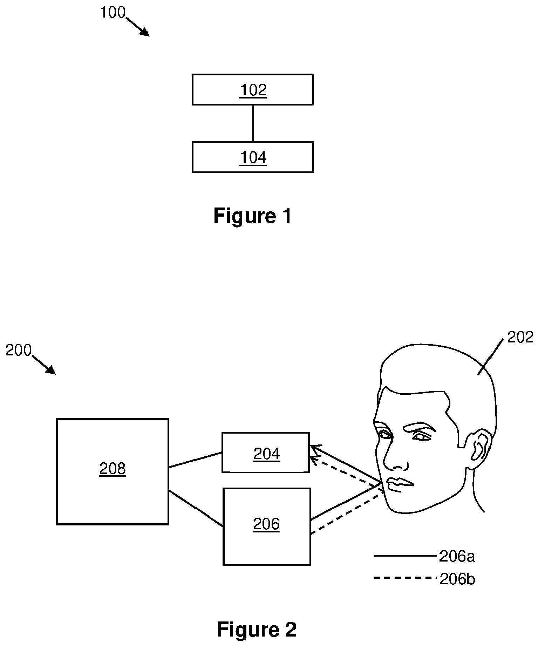

Fig. 1 shows a method 100 (e.g., a computer-implemented method) of determining certain information about a surface of a subject (e.g., the skin of a user). The method 100 may be implemented by a computer such as a user device, or a server or cloud-based service (e.g., communicatively coupled to the user device). An example of a user device includes a smart device such as a smartphone, tablet, smart mirror or any other device capable of processing imaging data as described below.

The method 100 comprises, at block 102, receiving imaging data.
As described in more detail below, the imaging data may be obtained by an imaging system (prior to being received according to block 102 of the method 100). The imaging data may refer to information such as pixel intensity information derived from at least one image obtained by the imaging system of a subject illuminated by first illumination and second illumination. The first illumination is in a first spectral band and the second illumination is in a second spectral band. The first spectral band comprises different spectral content to the first spectral band. The first spectral band may or may not overlap with the second spectral band. More details of the first and second spectral band are described in more detail below. The second illumination incident on the subject is polarized (e.g., linearly polarized).

The imaging system is configured to obtain first imaging data within the first spectral band by admitting at least part of the first spectral band into the imaging system and preventing admission of at least part of the second spectral band into the imaging system such that a majority of intensity information in the first imaging data obtained within the first spectral band is derived from the first illumination.

The imaging system is further configured to obtain second imaging data within the second spectral band by admitting at least part of the second spectral band into the imaging system and preventing admission of at least part of the first spectral band into the imaging system such that a majority of intensity information in the second imaging data obtained within the second spectral band is derived from the second illumination.

Thus, the imaging system may obtain both the first imaging data and the second imaging data, which may be distinct from the first imaging data in that different reflection information may be apparent in the first and second imaging data.

The first imaging data may correspond to imaging performed within the first spectral band. The second imaging data may correspond to imaging performed within the second spectral band. Depending on the spectral overlap between the channels used for obtaining the first and second imaging data, as well as the spectral overlap between the first spectral band and the second spectral band, the first imaging data may or may not comprise information derived from the second illumination, and vice versa.

Embodiments of the imaging system described herein may provide ways to ensure that the majority of intensity information (e.g., at least 50% of the sum of the pixel intensity values) in the first imaging data obtained within the first spectral band is derived from the first illumination. Similarly, such embodiments may provide ways to ensure that the majority of intensity information (e.g., at least 50% of the sum of the pixel intensity values from the image) in the second imaging data obtained within the second spectral band is derived from the second illumination.

In other words, at least 50% (i.e., the 'majority') of the total intensity information (e.g., sum of the pixel intensity values within the first imaging data) registered in the first imaging data is caused by the first illumination (for instance, 'red' light) being admitted into the imaging system and being detected by the pixels of an imaging device of the imaging system. Correspondingly, less than 50% (i.e., a 'minority') of the total intensity information (e.g., sum of the pixel intensity values within the first imaging data) registered in the first imaging data is caused by the second illumination (for instance, 'blue' light) being admitted into the imaging system and being detected by the pixels of an imaging device of the imaging system. Corresponding logic applies to the second imaging data.

The imaging system is further configured to admit the first and second illumination into the imaging system depending on a polarization state of reflected first and second illumination received by the imaging system after reflection from a surface of the subject such that specular and diffuse reflected first illumination is admitted into the imaging system and diffuse reflected second illumination is admitted into the imaging system.

As explained in more detail below, the first and second illumination incident on (i.e., directed towards) the subject has a certain initial polarization state (e.g., polarized or unpolarized). Upon reflection from the surface (and/or sub-surface) of the subject, this polarization state may or may not be retained. For example, diffusely reflected illumination may be unpolarized (or randomly polarized) irrespective of the polarization state of the illumination incident on the surface (e.g., the 'roughness' of the surface may at least partially randomize the polarization state upon reflection). However, specular reflected illumination may at least partially maintain the polarization state of the illumination incident on the surface if the illumination incident on the surface is initially polarized. Unpolarized illumination incident on the surface may remain unpolarized after specular reflection. However, in some cases, unpolarized illumination incident on the surface may be at least partially polarized upon reflection, depending on the angle of incidence.

The imaging system is configured such that specular and diffuse reflected illumination may or may not be admitted into the imaging system, depending on its polarization state upon entry to the imaging system (i.e., after reflection from the surface).

The method 100 further comprises, at block 104, determining information regarding specular reflection from the surface of the subject by comparing the first and second imaging data.

As noted above, the first imaging data comprises information regarding both specular and diffuse reflected first illumination. The second imaging data comprises information regarding diffuse reflected second illumination. Specular reflections may be indicative of the level of apparent gloss while diffuse reflections may obscure such specular reflections. By comparing the first and second imaging data, it may be possible to determine information regarding the specular reflections, and hence the level of gloss, since the comparison may decouple the specular reflection information from the diffuse reflection information.

Thus, in some embodiments, the information regarding specular information is indicative of a gloss level of the subject's skin. The gloss level may be determined by calculating a difference between intensity information in the first imaging data and the second imaging data. In other similar words, a result of the comparison between the first and second imaging data corresponds to a measure of glossiness of the subject's skin.

Certain embodiments described herein may facilitate (e.g., full-face) visualization of skin gloss in a relatively inexpensive manner. For example, a user device such as a smart phone equipped with an imaging system comprising an imaging device may be capable of acquiring the first and second imaging data. In some cases, additional equipment such as a hardware module (e.g., comprising a polarizer and/or illumination system) that can be coupled to the user device or used in situ with the user device may be used to facilitate obtaining the first and second imaging data (e.g., to admit first and second illumination depending on its polarization state, as referred to in the method 100 and/or provide the first and second illumination). In other cases, a dedicated device may have the hardware and corresponding functionality to facilitate obtaining the first and second imaging data, providing the first and second illumination and/or determining the information referred to in the method 100. In addition, certain embodiments described herein may facilitation the provision of precise and/or accurate measurement of the skin gloss and tracking/monitoring over time.

Fig. 2 shows a system 200 for determining certain information about a surface of a subject according to certain embodiments. The system 200 may at least partially implement certain methods described herein such as method 100 above. Certain blocks of the system 200 may be omitted in some embodiments.

The system 200 is being used by a subject 202 and comprises an imaging system 204 and an illumination system 206. The imaging system 204 is used to acquire imaging data referred to in the method 100. The illumination system 206 is configured to provide the first and second illumination 206a, 206b. The imaging system 204 and/or the illumination system 206 may be implemented by at least one device such as a user device. Thus, in some embodiments, separate devices may comprise the imaging system 204 and the illumination system 206 and, in other embodiments, the same device may comprise the imaging system 204 and the illumination system 206.

The system 200 further comprises a computer 208 (e.g., comprising processing circuitry implemented by a device or a server or cloud-based service for implementing certain methods described herein). Thus, the computer 208 may be communicatively coupled to the imaging system 204 and/or the illumination system 206 to send and/or receive data to/from these systems. This data may be processed by the processing circuitry of the computer 208 and/or be stored in a memory (e.g., of the computer 208 or accessible to processing circuitry of the computer 208). In some embodiments, the computer 208 may control the operation of the imaging system 204 and/or the illumination system 206. In some embodiments, the computer 208 comprises a controller for controlling illumination parameters (e.g., operating parameters for the illumination system 206) and/or detection parameters (e.g., operating parameters for the imaging system 204) and storing and/or processing the captured images or videos.

As depicted by Fig. 2, both the first illumination 206a (solid line) and the second illumination 206b (dashed line) is directed towards the surface of the subject 202 by the illumination system 206. The first and second illumination 206a, 206b incident on the subject is then reflected (e.g., specular and/or diffusely reflected) such that at least some of the reflected illumination 206a, 206b is directed towards and into the imaging system 204. As depicted by Fig. 2, the angle between the incident and reflected light depends on the respective positions of the imaging system 204 and illumination system 206 in relation to the subject 202. The operation of the system 200 may not strongly depend on the angle although different angles may affect how much illumination 206a, 206b is specular or diffuse reflected. In an embodiment, the angle of incidence is as close as possible to normal incidence (e.g., less than 10 degrees) although it shall be appreciated that other ranges of angles may be used depending on the configuration of the imaging system 204 and illumination system 206.

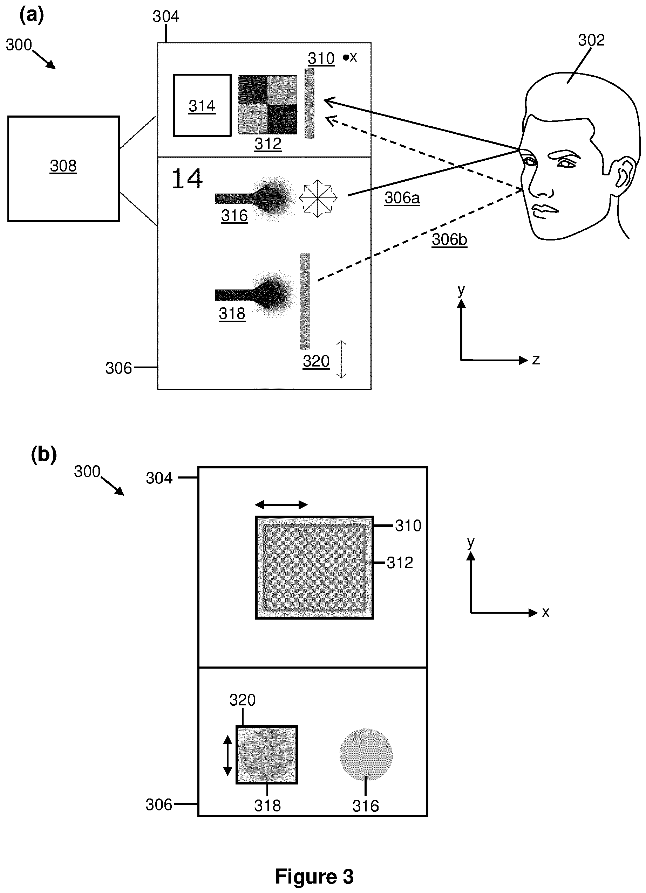

Fig. 3 shows different views (a) and (b) of a system 300 for determining certain information about a surface of a subject according to certain embodiments. View (a) is a side-view of the system 300 in a plane comprising the optical axis. View (b) is a front view of certain components of the system 300 in a plane perpendicular to the optical axis. Components of features of the system 300 are schematic and may be provided in any appropriate arrangement other than the one shown by Fig. 3. Reference numerals for features in system 300 with the same or similar functionality to corresponding features in system 200 are incremented by 100. The system 300 comprises certain corresponding features of the system 200 (i.e., an imaging system 304, illumination system 306 and the computer 308). The system 300 may at least partially implement certain methods described herein such as the method 100.

A description of the imaging system 304 is now given.

The imaging system 304 comprises a polarizing device (i.e., an 'imaging system polarizer' 310). The imaging system polarizer 310 is configured to admit, into the imaging system 304, reflected first and second illumination 306a, 306b (i.e., reflected from the surface of the subject 202) that has an electric field component that is parallel to a polarization axis of the imaging system polarizer 310; and attenuate reflected first and second illumination 306a, 306b that has an electric field component that is perpendicular to the polarization axis. The first and second illumination 306a, 306b that is attenuated upon admission into the imaging system 304 may be attenuated by absorption or reflection by the imaging system polarizer 310. The 'polarization axis' refers to the electric field vector direction that is selected for admission into the imaging system 304. For example, if the electric field vector of the illumination 306a, 306b is parallel to the polarization axis, the polarizing device admits this illumination 306a, 306b into the imaging system 304.

In some cases, the imaging system polarizer 310 comprises a (linear) polarizing device such as a polarizing filter, which may allow (at least partial) transmission of the first and second illumination 306a, 306b if the illumination 306a, 306b comprises a non-zero electric field (vector) component parallel to the polarization axis (which may also be referred to as a 'transmission axis') of the polarizing filter. The polarizing filter may attenuate transmission of the first and second illumination 306a, 306b if the illumination 306a, 306b comprises a non-zero electric field (vector) component perpendicular to the transmission axis of the polarizing filter.

In some cases, the imaging system polarizer 310 comprises a polarizing device such as a polarizing beam splitter (PBS, not shown) capable of transmitting or reflecting illumination depending on the polarization state of the incident illumination. Such a polarizing device may be configured to admit illumination of a certain polarization state into the imaging system 304 depending on the polarization state (e.g., polarized illumination reflected from the PBS may be admitted or polarized illumination transmitted by the PBS may be admitted depending on the configuration of the PBS). In any case, a polarization axis of the PBS may be oriented in such a way to select which polarization state is to be admitted into the imaging system 304.

Thus, a portion of the illumination 306a, 306b with a zero electric field vector component perpendicular to the polarization axis of the imaging system polarizer 310 (and non-zero component parallel to the polarization axis) is admitted into the imaging system 304 by the imaging system polarizer 310 with little or no attenuation.

However, a portion of the illumination 306a, 306b with a zero electric field vector component parallel to the polarization axis (and a non-zero component perpendicular to the polarization axis) is attenuated (potentially completely attenuated) by the imaging system polarizer 310. The level of attenuation thus depends on the ratio of the electric field vector components that are parallel and perpendicular to the polarization axis of the imaging system polarizer 310.

Thus, the imaging system polarizer 310 admits and/or attenuates the first and second illumination 306a, 306b reflected from the surface of the subject 302 depending on the polarization state of the reflected illumination 306a, 306b that is directed into the imaging system 304.

An optical axis ('z') may be defined between the imaging system 304 and the subject 302. The optical axis may also be referred to as an 'imaging axis'. In relation to the optical axis, z, the imaging system polarizer 310 has a 'horizontal' polarization axis in the 'x' direction depicted by the dot adjacent the imaging system polarizer 310 (where the corresponding 'y' direction is shown as vertical in Fig. 3). Thus, illumination 306a, 306b that has a non-zero electric field component in the x-axis is admitted into the imaging system 304 by the imaging system polarizer 310, although it may be attenuated depending on whether there is a non-zero electric field component perpendicular to the polarization axis of the imaging system polarizer 310. The coordinate system shown by Fig. 3 and elsewhere in this disclosure is provided to assist with explanation and other coordinate systems can be used.

In this embodiment, the imaging system polarizer 310 is configured to admit the first and second illumination 306a, 306b into the imaging system 304 depending on the polarization state of the reflected first and second illumination 306a, 306b received by the imaging system 304 after reflection from the surface of the subject 302 such that specular and diffuse reflected first illumination 306a is admitted into the imaging system 304 and diffuse reflected second illumination 306b is admitted into the imaging system 304. The illumination 306a, 306b admitted by the imaging system polarizer 310 (i.e., after admission) has a zero electric field component perpendicular to the polarization axis of the imaging system polarizer 310 (assuming a completely efficient polarizer, which is unlikely to be the case in reality).

In some embodiments, the specular and diffuse reflected first illumination 306a is attenuated depending on the polarization state of the reflected first illumination 306a incident on the subject 302. For example, approximately 50% of the total reflected first illumination 306a is admitted (the other 50% being attenuated due to absorption or reflection) if the polarization state of the reflected first illumination 306a is random. However, if the reflected first illumination 306a is not randomly polarized, the level of attenuation may vary depending on the angle between the polarization state of the first illumination 306a and the polarization axis. As a result, the first imaging data may comprise information regarding both specular and diffuse reflected first illumination 306a.

In addition, the specular reflected second illumination 306b may likely be almost completely attenuated if it has zero electric field vector component parallel to the polarization axis of the imaging system polarizer 310. The polarized second illumination 306b (before reflection) may have an electric field component that is perpendicular to the polarization axis of the imaging system polarizer 310. Providing the polarization state is maintained (and not substantially rotated) after reflection (i.e., it is not randomized due to surface roughness), the reflected second illumination 306b has a zero electric field component parallel to the polarization axis of the imaging system polarizer 310. As a result in this case, the second illumination 306b may be substantially attenuated such that not much or any second illumination 306b is admitted by the imaging system polarizer 310. In the case of surface roughness or low gloss, some diffuse reflection of the second illumination 306b may occur, which at least partially randomizes the polarization state of the (initially polarized) second illumination 306b. Thus, at least some of the diffused reflected second illumination 306b may have a non-zero electric field component aligned with the polarization axis, to allow at least attenuated admission of said diffused reflected second illumination 306b. Thus, the second imaging data may comprise information regarding diffuse reflected second illumination 306b (but not specular reflected second illumination 306b).

The imaging system polarizer 310 may therefore be used to admit and/or attenuate illumination 306a, 306b comprising certain polarization states. That is, the orientation of the polarization axis of the imaging system polarizer 310 is such that certain portions of the reflected first and second illumination 306a, 306b may be admitted or attenuated depending on the polarization state of the reflected first and second illumination 306a, 306b.

As highlighted above, the reflected first and second illumination 306a, 306b may have multiple polarization states due to the polarization state of different portions of the reflected first and second illumination 306a, 306b being modified by different extents after reflection from different surface types. For example, polarized second illumination 306b incident on a rough surface may become randomly polarized after reflection while polarized second illumination 306b incident on a smooth, glossy surface may substantially maintain its polarized state after reflection (i.e., initially polarized illumination may remain polarized after reflection).

In some embodiments, initially unpolarized (or randomly polarized) first illumination 306a may remain unpolarized after reflection from a rough surface. In some embodiments, initially unpolarized (or randomly polarized) first illumination 306a may be at least partially polarized after reflection from a smooth, or glossy surface. In some embodiments the first illumination 306a may be polarized with an initial polarization state that is orthogonal to the polarization state of the second illumination 306b.

Accordingly, in the case of unpolarized first illumination 306a and polarized second illumination 306b incident on the subject 302 that is smooth and/or glossy, the first imaging data (corresponding to imaging using the first illumination 306a) comprises information regarding both specular and diffuse reflected first illumination 306a. The registered pixel intensity levels for the second imaging data (corresponding to imaging using the second illumination 306b) may be much lower (i.e., darker) than for the first imaging data because the reflected second illumination 306b admitted into the imaging system may be substantially attenuated by the imaging system polarizer 310 due its zero or near-zero electric field component parallel to the polarization axis of the imaging system polarizer 310.

Thus, by calculating a difference between the first imaging data (comprising both specular and diffuse reflection information) and the second imaging data (comprising diffuse reflection information), information regarding the specular reflection information may be determined. In the above case, the level of diffuse reflection may be low and the level of specular reflection is relatively high due to the glossy surface.

In the case of unpolarized first illumination 306a and polarized second illumination 306b incident on the subject 302 that is rough, the first imaging data (corresponding to imaging using the first illumination 306a) comprises information regarding both specular and diffuse reflected first illumination 306a. The registered pixel intensity levels for the second imaging data (corresponding to imaging using the second illumination 306b) may not be dark (in contrast to the previous case) because the reflected second illumination 306b admitted into the imaging system 304 may not be substantially attenuated by the imaging system polarizer 310 (due its non-zero electric field component parallel to the polarization axis of the imaging system polarizer 310 resulting from the reflected second illumination 306b comprising diffuse reflections, which may be at least partially randomly polarized).

Thus, by calculating a difference between the first imaging data (comprising both specular and diffuse reflection information) and the second imaging data (comprising diffuse reflection information), information regarding the specular reflection information may be determined. In the above case, the level of diffuse reflection may be high and the level of specular reflection is relatively low due to the rough surface.

Therefore, the ratio of (or difference between) pixel intensity values in the first and second imaging data may be indicative of the relative amount of specular and diffuse reflections from the surface. If the second imaging data is relatively 'dark' (with low registered pixel intensity values) compared with the first imaging data, this may indicate a glossy surface. However, if the second imaging data is relatively 'bright' (with relatively higher registered pixel intensity values compared with the 'glossy' case) compared with the first imaging data, this may indicate a rough surface.

In some cases, the ratio or difference between the pixel intensity values in the first and second imaging data may provide a quantitative measurement of the specular reflection.

In some embodiments, the imaging system 304 comprises a color filter array 312 configured to enable at least one imaging device 314 of the imaging system 304 to obtain the first imaging data within the first spectral band and the second imaging data within the second spectral band. The color filter array 312 may be provided as part of the imaging device(s) 314 itself (e.g., a Bayer filter or another type of filter layer) or as a separate component.

In an embodiment, the color filter array 312 may be aligned with the pixels (not shown) of the imaging device 314 such that each filter 'cell' or 'channel' of the array 312 is aligned with a corresponding pixel of the imaging device 314. For example, a Bayer filter may comprise red, green and blue bandpass filter 'cells' which are aligned with the pixels of the imaging device 314. Thus, some pixels of the imaging device 314 register 'red' light, some pixels register 'green' light and the remaining pixels register 'blue' light. In an embodiment, the 'red' pixels may provide first imaging data and the 'green' pixels may provide the second imaging data. In another embodiment, the 'red' pixels may provide the first imaging data and the 'blue' pixels may provide the second imaging data. Any other combination of colors associated with the pixels may provide the first and second imaging data.

Thus, in some embodiments, the imaging system 304 comprises at least one imaging device 314 and an optical filter array 312. The optical filter array 312 is configured to pass, to a first set of pixels of the at least one imaging device 314, at least part of the first spectral band into the imaging system 304 such that the majority of intensity information in the first imaging data obtained within the first spectral band is derived from the first illumination 306a. The optical filter array 312 is further configured to pass, to a second, different, set of pixels of the at least one imaging device 314, at least part of the second spectral band into the imaging system 304 such that the majority of intensity information in the second imaging data obtained within the second spectral band is derived from the second illumination 306a.

The imaging device(s) 314 and/or the computer 308 may extract, from raw imaging data obtained by the imaging device(s) 314, the first imaging data separately from the second imaging data. For example, certain pixels of the imaging device(s) 314 which correspond to a certain color (e.g., one of red, green or blue) may provide pixel intensity information as 'raw' data for the first imaging data. Certain other pixels of the imaging device(s) 314 which correspond to another color (e.g., another one of red, green or blue) may provide pixel intensity information as 'raw' data for the second imaging data.

In another embodiment, the imaging system 304 comprises multiple (e.g., two or more) imaging devices (not shown). A first of the imaging devices may receive the first illumination but not the second illumination (e.g., via an appropriate optical arrangement including an appropriate optical filter). A second of the imaging devices may receive the second illumination but not the first illumination. Thus, the first imaging device may provide the first imaging data and the second imaging device may provide the second imaging data.

According to the arrangements described above, the first and second imaging data can be obtained over the same or an overlapping time period. In other words, in some embodiments, a timeframe over which the first imaging data is obtained at least partially overlaps with a timeframe over which the second imaging data is obtained. This may occur where certain pixels (or a first imaging device) associated with the first imaging data performs imaging (e.g., acquires a frame) over a first time period and certain pixels (or a second imaging device) associated with the second imaging data performs imaging over a second time period which at least partially overlaps with the first time period.

In another embodiment, the imaging system 304 is configured to simultaneously obtain the first and second imaging data. For example, pixels configured to measure intensity levels for the first imaging data may measure such intensity levels at the same time as pixels configured to measure intensity levels for the second imaging data.

By obtaining the first and second imaging data over an overlapping time period and/or simultaneously, the imaging data for determining the information regarding specular reflection may be obtained in a manner that is relatively straightforward and inexpensive. For example, a user device such as a smartphone may be configured to obtain the first and second imaging data in a straightforward manner by providing an imaging system polarizer in conjunction with the imaging device(s) of the user device as described above. In some cases, an imaging system polarizer 310 may be a component of the user device itself or, in other cases, a separate component.

As the surface of the subject (e.g., a user's face) may move voluntarily and/or involuntarily, obtaining the first and second imaging data over an overlapping time period and/or simultaneously may reduce error when determining the information regarding specular information. For example, the same pixels may be more likely to register information from the same part of the surface of the user's face if the first and second imaging data is obtained simultaneously. Further, in some cases, gloss measurements may be taken of multiple views of the face to cover multiple angles. Embodiments described herein may facilitate taking a sequence of frames with an imaging device (e.g., a video or sequence of images) that is capable of spectrally separating the first and second imaging data simultaneously.

The spectral gating of the first illumination and second illumination, along with the polarization dependency of illumination 306a, 306b admitted into the imaging system 304 may allow the first imaging data to be distinguished from the second imaging data. Thus, the configuration of the imaging system 304 and the illumination system 306 may provide a way to easily distinguish between reflected illumination 306a, 306b which comprises specular reflection information from reflected illumination 306a, 306b which comprises diffuse reflection information, and hence determine the specular reflection information.

A description of the illumination system 306 is now given.

The illumination system 306 comprises a first illumination source 316 (e.g., at least one light-emitting diode (LED) or other type of light source) configured to provide the first illumination 306a. In this embodiment, the first illumination 306a is unpolarized and is directed towards the subject 302. The illumination system 306 further comprises a second illumination source 318 (e.g., at least one light-emitting diode (LED) or other type of light source) configured to provide the second illumination 306b. The second illumination 306a is directed towards the subject 302 in a similar manner to the first illumination 306b. In this regard, the first illumination source 316 and second illumination source 318 may be adjacent to each other or in any appropriate arrangement to illuminate the subject 302.

The illumination system 306 comprises an illumination system polarizer 320 configured to polarize the second illumination 306b that is directed towards the subject 302. A polarization axis of the imaging system polarizer 310 is orthogonal to the polarization axis of the illumination system polarizer 320 (e.g., with respect to the optical axis). In other similar words, the polarization states admitted or transmitted by the imaging system polarizer 310 and illumination system polarizer 320 may be orthogonal to each other (or the two orthogonal polarization states may represent diagonally opposite points on the Poincare sphere). As shown by Fig. 3, the imaging system polarizer 310 has a polarization axis in the x-direction and the illumination system polarizer 320 has a polarization axis in the y-direction.

In some embodiments, the illumination system 306 is configured to direct the first and second illumination 306a, 306b towards the subject 302 such that both a specular and diffuse component of the first and second illumination 306a, 306b reflected from the surface of the subject 302 is directed into the imaging system 304 for admission thereinto depending on the polarization state of the reflected first and second illumination 306a, 306b.

As noted above, the angle of incidence may vary depending on the configuration. In a possible configuration, the illumination system 306 comprises the first and second illumination sources 316, 318 positioned adjacent to the imaging system 304 such that the angle of incidence is as close as possible to normal incidence. In a possible configuration, multiple first and second illumination sources 316, 318 may be positioned around (e.g., concentrically around in a ring) the imaging system 304 so that the subject 302 is illuminated evenly from multiple angles.

In the embodiment shown by Fig. 3, the illumination system 306 is configured such that the first illumination 306a directed towards the subject 302 is unpolarized. However, in some embodiments, the illumination system 306 comprises an additional illumination system polarizer (not shown) configured to polarize the first illumination 306a directed towards the subject 302 such that the polarization state of the first illumination 306a incident on the subject 302 is orthogonal to the polarization state of the second illumination 306b incident on the subject. In other words, the additional illumination system polarizer may have its polarization axis oriented along the x-axis in Fig. 3.

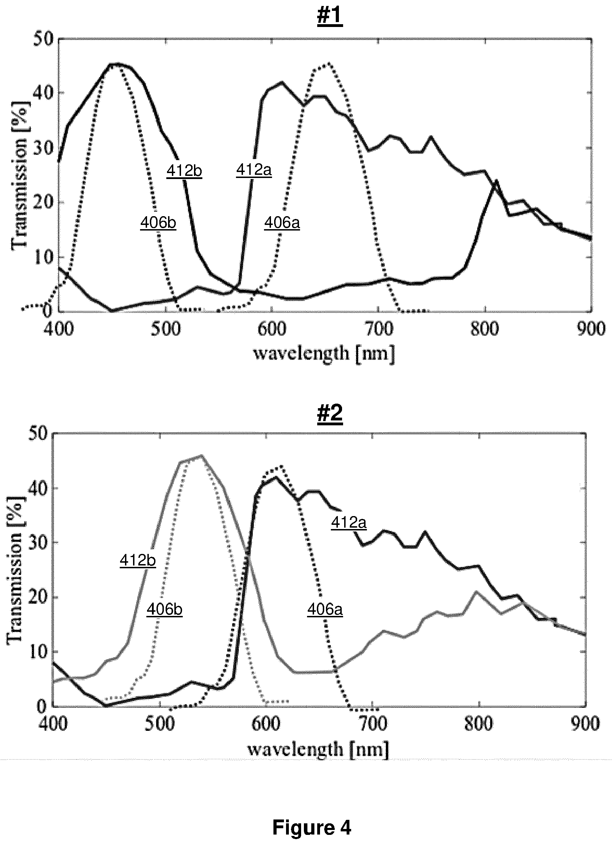

Fig. 4 shows example optical parameters of certain components of the system 300 of Fig. 3 in graph form of transmission (%) as function of wavelength (for the solid lines) and is overlaid with dotted lines corresponding to the spectral content of the first and second illumination sources 316, 318 referred to in Fig. 3. Reference numerals for features referred to in Fig. 3 are incremented by 100.

In case #1, the first illumination 406a (dotted line) comprises spectral content in a first spectral band centered around 650 nm (nanometers), i.e., towards the 'red' part of the spectrum. The full-width half-maximum spectral width of the first spectral band is around 60 nm. The color filter array 312 of Fig. 3 comprises different 'cells' for admitting/passing the first spectral band. In Fig. 4, the spectral admission band for admitting the first illumination 406a is depicted by solid line 412a. The spectral admission band is predominantly in the 'red' to 'infrared' part of the spectrum (e.g., between around 570 to 900 nm). The first spectral band of the first illumination 406a substantially overlaps with the spectral admission band 412a. Thus, the cells in the color filter array 312 of Fig. 3 that correspond to the spectral admission band 412a admit the first illumination 406a such that the first illumination 406a is detected by the corresponding pixels (e.g., to reduce detecting illumination in other spectral bands).

The second illumination 406b (dotted line) comprises spectral content in a second spectral band centered around 450 nm, i.e., towards the 'blue' part of the spectrum. The full-width half-maximum spectral width of the second spectral band is around 50 nm. The spectral admission band for admitting the second illumination 406b is depicted by solid line 412b. The spectral admission band is predominantly in the 'bluer' part of the spectrum (e.g., between <400 to 550 nm). The second spectral band of the second illumination 406b substantially overlaps with the spectral admission band 412b. Thus, the cells in the color filter array 312 of Fig. 3 that correspond to the spectral admission band 412b admit the second illumination 406b such that the second illumination 406b is detected by the corresponding pixels (e.g., to reduce detecting illumination in other spectral bands such as the first illumination 406a).

In case #2 of Fig. 4, the first illumination 406a and spectral admission band 412a is the same as case #1. However, the second illumination 406b (dotted line) comprises spectral content in a second spectral band centered around 530 nm, i.e., comprising the 'green' part of the spectrum. The full-width half-maximum spectral width of the second spectral band is around 50 nm. The spectral admission band for admitting the second illumination 406b is depicted by solid line 412b. The spectral admission band is predominantly in the 'green' part of the spectrum (e.g., between around 450 to 610 nm). The second spectral band of the second illumination 406b substantially overlaps with the spectral admission band 412b. Thus, the cells in the color filter array 312 of Fig. 3 that correspond to the spectral admission band 412b admit the second illumination 406b such that the second illumination 406b is detected by the corresponding pixels (to reduce detecting illumination in other spectral bands such as the first illumination 406a).

Case #2 highlights how there may be a degree of spectral overlap between the spectral admission bands and/or first and second illumination 406a, 406b providing there is sufficient spectral distinction as discussed below.

This spectral distinction may be obtained if the wavelength bands (e.g., intervals) of each illumination source 316, 318 have: 1) substantial spectral overlap with the spectral sensitivity of the associated channel (e.g., substantial overlap of the spectral content of the illumination 406a, 406b with the corresponding spectral admission bands 412a, 412b) of the imaging system 304 (e.g., a red light source may have an associated red detection channel); and 2) minimal spectral overlap with the spectral sensitivity of the non-associated channel/s. In other similar words, a majority of intensity information in the first imaging data obtained within the first spectral band is derived from the first illumination 406a. Similarly, a majority of intensity information in the second imaging data obtained within the second spectral band is derived from the second illumination 406b. To a certain extent in case #1 and more so in case #2, there may be some 'leakage' between the channels. However, the majority of the pixel intensity information is likely to be derived from the correct channel due to the spectral distinction described above.

Case #1 refers to a scenario which may be regarded as having an ideal overlap (e.g., overlap is measured as the ratio of the overlapping area under the curve to the area of the illumination source spectrum) between the first illumination source 406a and the corresponding detection channel defined by the spectral admission band 412a. The same applies for the second illumination source 406b and the corresponding detection channel defined by the spectral admission band 412b. Here, the spectral overlap between the first and second illumination 406a, 406b and the non-associated detection channel (e.g., the second and first spectral admission bands 412b, 412a, respectively) is minimal, e.g., <10% of the total spectral width is overlapped. It may be observed that the spectral overlap between the first and second illumination 406a, 406b and the non-associated detection channel spectra is roughly equivalent to the error in the calculated specular reflection. In case #2, there may be a greater degree of overlap compared with case #1 but there is sufficient spectral distinction to facilitate the spectral gating operation.

The choice of wavelength or wavelengths for each of the first and second illumination 406a, 406b is generally not relevant, except for being appropriate for the wavelength detection range of the associated detection channel(s). For example, rather than having the first illumination 406a comprising the red part of the spectrum and the second illumination 406b comprising the blue or green part of the spectrum as in cases #1 and #2 above, another configuration may be selected. For example, the first illumination 406a may comprise the blue or green part of the spectrum and the second illumination 406b may comprise the green or red part of the spectrum. Further, non-visible illumination sources, e.g., ultraviolet or infrared, may be used for at least one of the illumination sources.

Thus, with reference also to Fig. 3 and in some embodiments, the illumination system 306 may comprise a first illumination unit (e.g., the first illumination source 316) for providing light illumination (e.g., directed to the face of a subject 302). The first illumination unit provides first illumination 406a by emitting at a first spectral emission band. The first spectral emission band is selected to be substantially overlapping a first spectral detection band of an imaging unit (e.g., the imaging system 304)

The illumination system 306 may comprise a second illumination unit (e.g., the second illumination source 318) for providing linearly polarized light (illumination) (e.g., directed to the face of the subject 302). The second illumination unit provides second illumination 406b by emitting at a second spectral emission band. The second spectral emission band is selected to be substantially overlapping a second spectral detection band of the imaging unit.

A first linear polarizing filter (e.g., illumination system polarizer 320) associated with the second illumination unit is oriented perpendicular to the polarization axis of light admitted into the imaging unit.

The imaging unit comprises a second linear polarizing filter (e.g., imaging system polarizer 310). The imaging unit is configured to admit at least two spectral detection bands. The imaging unit is configured to capture (e.g., image) diffuse reflectance of the second illumination 406b reflected from the surface (e.g., face) of a subject 302. The imaging unit is further configured to capture total (e.g., diffuse and specular) reflectance of the first illumination 406a reflected by the surface of the same subject. The wavelength and the bandwidth of the illumination 406a, 406b is decided in combination with the spectral detection bands associated with the imaging unit.

A control unit (e.g., computer 308) may be provided for controlling the illumination and detection parameters and storing and/or processing the captured RAW Bayer images or videos (e.g., receiving the first and second imaging data and determining information regarding specular reflection). As noted above, specular reflection may be extracted from the two separate channels from the RAW Bayer image data.

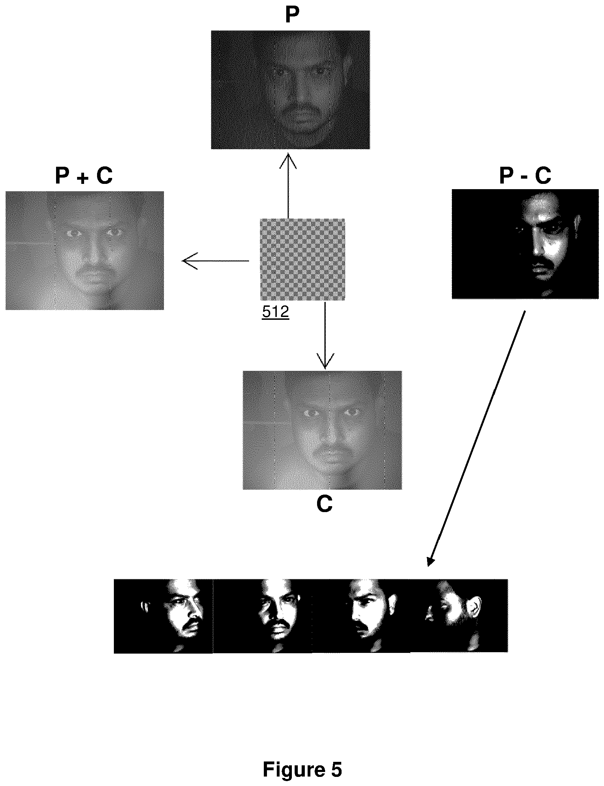

Fig. 5 is a schematic drawing of a representation of the method of determining certain information about a surface of a subject according to an embodiment. Reference is made to features of Fig. 3 and reference numerals for like features are incremented by 200. The representation shows the imaging data (indicated by images) obtained by the procedure described above.

According to Fig. 5, the information regarding specular and diffuse reflection is shown in the parallel 'P' image, which is derived from the admitted first illumination 306a. The information regarding diffuse reflection is shown in the cross 'C' image, which is derived from the admitted second illumination 306b. The total intensity is shown by the 'P+C' image. The difference between the 'P' and 'C' image is shown by the 'P-C' image. Thus, the 'P-C' image corresponds to the specular reflected image. Each of the images is derived by 'de-Bayering' or extracting the individual channels (i.e., red, blue or green) admitted by the corresponding pixels related to the color filter array 512. Fig. 5 also shows a sequence of frames obtained at different angles of the specular reflected image. The process for obtaining each frame is relatively straightforward (e.g., in some cases, one frame is acquired at each angle and just that one frame is needed to extract the specular reflection information).

Some commercial color cameras, such as those of mobile phones, have an in-built Bayer filter (e.g., comprising a 2 green, a red and a blue sub-filter). By utilizing the spectral gating procedure and polarization gating by orthogonal polarizers as described herein, cross and parallel polarized images of the face may be obtained simultaneously without any motion artefact. It may therefore be possible to obtain the cross and parallel polarized image simultaneously, which may reduce cost and/or complexity, and permit rapid acquisition and processing of frames e.g., for multiple frames at different angles.

Due to the scattering and reflection property of skin, the combination of a color camera and multiple illumination sources, of which at least one is polarized, has less overlap in the Bayer spectrum. This arrangement may provide two images (e.g., corresponding the first and second imaging data) of which one comprises specular reflections and other without any specular reflections (i.e., diffuse reflection instead).

The intensity recorded in the two images may be distinct due to different absorption, scattering and polarization properties of the skin (as well as different intensity of the first and second illumination 306a, 306b provided by the illumination system 306). The sensitivity of the gloss measurement can be refined by selecting appropriate wavelengths of light by prior knowledge of the Bayer filters (or other type of color filter array 512 or other optical arrangement) and the specular reflection spectrum of the skin.

Further, certain methods described herein may be extended by analyzing the histogram of the pixel intensity information in the 'P-C' image to quantitatively measure e.g., oiliness reduction after a personal care regime, etc. For example, a P-C image histogram with a peak towards higher pixel intensity values may indicate lots of specular reflection whereas a more even distribution of pixel intensity values (or a peak towards lower pixel intensity values) may indicate minimal specular reflection.

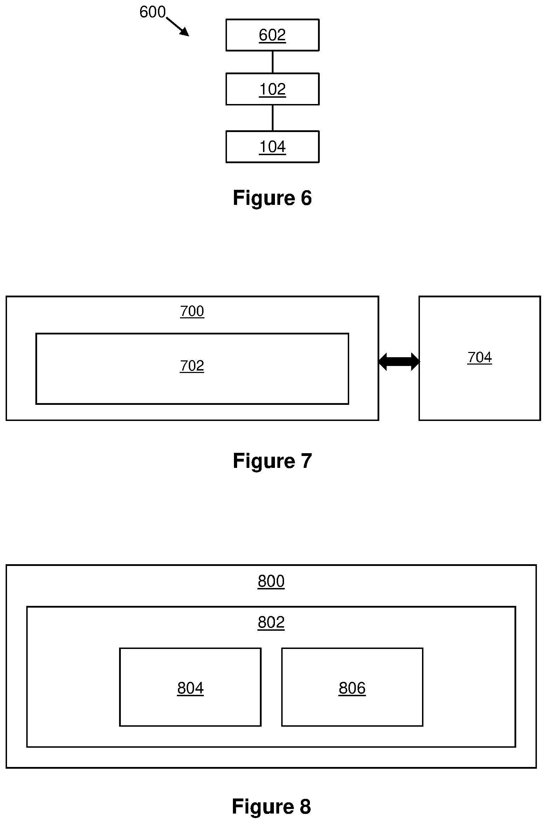

Fig. 6 shows a method 600 (e.g., a computer-implemented method) of determining certain information about a surface of a subject (e.g., the skin of a user). The method 600 may be implemented by a computer such as a user device, or a server or cloud-based service (e.g., communicatively coupled to the user device). An example of a user device includes a smart device such as a smartphone, tablet, smart mirror or any other device capable of processing imaging data as described below. Reference is made to Fig. 3 in the description below.

The method 600 comprises the blocks 102 and 104 of the method 100. The method 600 further comprises extracting, at block 602, from raw imaging data obtained by the at least one imaging device 314, the first imaging data separately from the second imaging data. Block 602 is implemented prior to block 102.

Fig. 7 shows a tangible machine-readable medium 700 storing instructions 702 which, when executed by processing circuitry (e.g., at least one processor) 704, cause the processing circuitry 704 to implement certain methods described herein, such as method 100, method 600 and/or related embodiments.

Fig. 8 shows an apparatus 800, which may be used for implementing certain methods described herein such as the method 100, method 600 and/or related embodiments. The apparatus 800 may comprise modules with functionality corresponding to certain features described in relation to the system 200 of Fig. 2 such as the computer 208 thereof. The apparatus 800 comprises processing circuitry 802.

The processing circuitry 802 comprises a receiving module 804 configured to receive imaging data obtained by an imaging system 204 of a subject 202. The subject 202 is illuminated by first illumination 206a in a first spectral band and second illumination 206b in a second spectral band comprising different spectral content to the first spectral band. The second illumination 206a incident on the subject is polarized.

The imaging system 204 is configured to obtain first imaging data within the first spectral band by admitting at least part of the first spectral band into the imaging system and preventing admission of at least part of the second spectral band into the imaging system such that a majority of intensity information in the first imaging data obtained within the first spectral band is derived from the first illumination 206a.

The imaging system 204 is further configured to obtain second imaging data within the second spectral band by admitting at least part of the second spectral band into the imaging system and preventing admission of at least part of the first spectral band into the imaging system such that a majority of intensity information in the second imaging data obtained within the second spectral band is derived from the second illumination 206b.

The imaging system 204 is further configured to admit the first and second illumination into the imaging system 204 depending on a polarization state of reflected first and second illumination received by the imaging system after reflection from a surface of the subject such that specular and diffuse reflected first illumination 206a is admitted into the imaging system 204 and diffuse reflected second illumination 206b is admitted into the imaging system 204.

The processing circuitry 802 further comprises a determining module 806 configured to determine information regarding specular reflection from the surface of the subject 202 by comparing the first and second imaging data.

In some embodiments, the apparatus 800 further comprises the imaging system 204. In some embodiments, the apparatus 800 further comprises the illumination system 206 configured to provide the first and second illumination 206a, 206b.

In some cases, any of the modules described above (e.g., the receiving module 804 and/or the determining module 806) may comprise at least one dedicated processor (e.g., an application specific integrated circuit (ASIC) and/or field programmable gate array (FPGA), etc.) for implementing the functionality of the module.

In some cases, the module above (e.g., the receiving module 804 and/or the determining module 806) may comprise at least one processor for implementing instructions which cause the at least one processor to implement the functionality of the module described above. In such examples, the instructions may be stored in a machine-readable medium (not shown) accessible to the at least one processor. In some examples, the module itself comprises the machine-readable medium. In some examples, the machine-readable medium may be separate to the module itself (e.g., the at least one processor of the module may be provided in communication with the machine readable medium to access the instructions stored therein).

While the invention has been illustrated and described in detail in the drawings and foregoing description, such illustration and description are to be considered illustrative or exemplary and not restrictive; the invention is not limited to the disclosed embodiments.

One or more features described in one embodiment may be combined with or replace features described in another embodiment. For example, the methods 100, 600 of Figs. 1 and 6 may be modified based on features described in relation to the systems 200, 300 of Figs. 2 and 3, the machine-readable medium 700 and/or the apparatus 800, and vice versa.

Embodiments in the present disclosure can be provided as methods, systems or as a combination of machine-readable instructions and processing circuitry. Such machine-readable instructions may be included on a non-transitory machine (for example, computer) readable storage medium (including but not limited to disc storage, CD-ROM, optical storage, etc.) having computer readable program codes therein or thereon.

The present disclosure is described with reference to flow charts and block diagrams of the method, devices and systems according to embodiments of the present disclosure. Although the flow charts described above show a specific order of execution, the order of execution may differ from that which is depicted. Blocks described in relation to one flow chart may be combined with those of another flow chart. It shall be understood that each block in the flow charts and/or block diagrams, as well as combinations of the blocks in the flow charts and/or block diagrams can be realized by machine readable instructions.

The machine readable instructions may, for example, be executed by a general purpose computer, a special purpose computer, an embedded processor or processors of other programmable data processing devices to realize the functions described in the description and diagrams. In particular, a processor or processing circuitry, or a module thereof, may execute the machine readable instructions. Thus functional modules of the computer 208, computer 308 and/or apparatus 800 (for example, the receiving module 804 and/or the determining module 806) and other devices described herein may be implemented by a processor executing machine readable instructions stored in a memory, or a processor operating in accordance with instructions embedded in logic circuitry. The term 'processor' is to be interpreted broadly to include a CPU, processing unit, ASIC, logic unit, or programmable gate array etc. The methods and functional modules may all be performed by a single processor or divided amongst several processors.

Such machine readable instructions may also be stored in a computer readable storage that can guide the computer or other programmable data processing devices to operate in a specific mode.

Such machine readable instructions may also be loaded onto a computer or other programmable data processing devices, so that the computer or other programmable data processing devices perform a series of operations to produce computer-implemented processing, thus the instructions executed on the computer or other programmable devices realize functions specified by block(s) in the flow charts and/or in the block diagrams.

Further, the teachings herein may be implemented in the form of a computer program product, the computer program product being stored in a storage medium and comprising a plurality of instructions for making a computer device implement the methods recited in the embodiments of the present disclosure.

Elements or steps described in relation to one embodiment may be combined with or replaced by elements or steps described in relation to another embodiment. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method (100), comprising:
receiving (102) imaging data obtained by an imaging system (204) of a subject (202) illuminated by first illumination (206a) in a first spectral band and second illumination (206b) in a second spectral band comprising different spectral content to the first spectral band, wherein the second illumination incident on the subject is polarized, and wherein the imaging system is configured to:
obtain first imaging data within the first spectral band by admitting at least part of the first spectral band into the imaging system and preventing admission of at least part of the second spectral band into the imaging system such that a majority of intensity information in the first imaging data obtained within the first spectral band is derived from the first illumination;
obtain second imaging data within the second spectral band by admitting at least part of the second spectral band into the imaging system and preventing admission of at least part of the first spectral band into the imaging system such that a majority of intensity information in the second imaging data obtained within the second spectral band is derived from the second illumination; and
admit the first and second illumination into the imaging system depending on a polarization state of reflected first and second illumination received by the imaging system after reflection from a surface of the subject such that specular and diffuse reflected first illumination is admitted into the imaging system and diffuse reflected second illumination is admitted into the imaging system; and
determining (104) information regarding specular reflection from the surface of the subject by comparing the first and second imaging data.

2. The method of claim 1, wherein a timeframe over which the first imaging data is obtained at least partially overlaps with a timeframe over which the second imaging data is obtained.

3. The method of claim 2, wherein the imaging system is configured to simultaneously obtain the first and second imaging data.

4. The method of any of claims 1 to 3, wherein the imaging system (304) comprises an imaging system polarizer (310) configured to admit, into the imaging system, reflected first and second illumination (306a, 306b) that has an electric field component that is parallel to a polarization axis of the imaging system polarizer; and attenuate reflected first and second illumination that has an electric field component that is perpendicular to the polarization axis.

5. The method of any of claims 1 to 4, wherein the information regarding specular information is indicative of a gloss level of the subject's skin, and wherein the gloss level is determined by calculating a difference between intensity information in the first imaging data and the second imaging data.

6. The method (600) of any of claims 1 to 5, wherein the imaging system (304) comprises a color filter array (312) configured to enable at least one imaging device (314) of the imaging system to obtain the first imaging data within the first spectral band and the second imaging data within the second spectral band, the method further comprising extracting (602), from raw imaging data obtained by the at least one imaging device, the first imaging data separately from the second imaging data.

7. A tangible machine-readable medium (700) comprising instructions (702) which, when executed by processing circuitry (704), causes the processing circuitry to implement the method of any of claims 1 to 6.

8. Apparatus (208, 800) comprising processing circuitry (802), the processing circuitry comprising:
a receiving module (804) configured to receive (102) imaging data obtained by an imaging system (204) of a subject (202) illuminated by first illumination (206a) in a first spectral band and second illumination (206b) in a second spectral band comprising different spectral content to the first spectral band, wherein the second illumination incident on the subject is polarized, and wherein the imaging system (204) is configured to:
obtain first imaging data within the first spectral band by admitting at least part of the first spectral band into the imaging system and preventing admission of at least part of the second spectral band into the imaging system such that a majority of intensity information in the first imaging data obtained within the first spectral band is derived from the first illumination;
obtain second imaging data within the second spectral band by admitting at least part of the second spectral band into the imaging system and preventing admission of at least part of the first spectral band into the imaging system such that a majority of intensity information in the second imaging data obtained within the second spectral band is derived from the second illumination; and
admit the first and second illumination into the imaging system depending on a polarization state of reflected first and second illumination received by the imaging system after reflection from a surface of the subject such that specular and diffuse reflected first illumination is admitted into the imaging system and diffuse reflected second illumination is admitted into the imaging system; and
a determining module (806) configured to determine (104) information regarding specular reflection from the surface of the subject by comparing the first and second imaging data.

9. The apparatus of claim 8, wherein a result of the comparison between the first and second imaging data corresponds to a measure of glossiness of the subject's skin.

10. The apparatus of any of claims 8 to 9, wherein the apparatus further comprises the imaging system (204) and/or an illumination system (206) configured to provide the first and second illumination (206a, 206b).

11. The apparatus of claim 10, wherein the imaging system (304) comprises an imaging system polarizer (310) configured to admit, into the imaging system, reflected first and second illumination (306a, 306b) that has an electric field component that is parallel to a polarization axis of the imaging system polarizer; and prevent admission, into the imaging system, of reflected first and second illumination that has an electric field component that is perpendicular to the polarization axis.

12. The apparatus of claim 11, wherein the illumination system comprises an illumination system polarizer (320) configured to polarize the second illumination that is directed towards the subject, wherein a polarization axis of the imaging system polarizer is orthogonal to the polarization axis of the illumination system polarizer.

13. The apparatus of any of claims 10 to 12, wherein the illumination system is configured such that the first illumination directed towards the subject is unpolarized or the illumination system comprises an additional illumination system polarizer configured to polarize the first illumination directed towards the subject such that the polarization state of the first illumination that is directed towards the subject is orthogonal to the polarization state of the second illumination that is directed towards the subject.

14. The apparatus of any of claims 10 to 13, wherein the illumination system is configured to direct the first and second illumination towards the subject such that both a specular and diffuse component of the first and second illumination reflected from the surface of the subject is directed into the imaging system for admission thereinto depending on the polarization state of the reflected first and second illumination.

15. The apparatus of any of claims 10 to 14, wherein the imaging system (304) comprises at least one imaging device (314) and an optical filter array (312), wherein the optical filter array is configured to:
pass, to a first set of pixels of the at least one imaging device, at least part of the first spectral band into the imaging system such that the majority of intensity information in the first imaging data obtained within the first spectral band is derived from the first illumination; and
pass, to a second, different, set of pixels of the at least one imaging device, at least part of the second spectral band into the imaging system such that the majority of intensity information in the second imaging data obtained within the second spectral band is derived from the second illumination.
